# EUROPEAN PATENT APPLICATION

(11) **EP 2 177 905 A1**
(43) Date of publication of application: **21.04.2010**
(21) Application number: 08805295.6
(22) Date of filing: 30.06.2008
(51) Int. Cl.: G01N 33/18, G01N 21/64, G01N 21/77

(54) **BIOSENSORS BASED ON MICROALGAE FOR DETECTION OF ENVIRONMENTAL POLLUTANTS**

(30) Priority: 03.07.2007 ES 200701905
(71) Applicant: Universidad Complutense de Madrid Rectorado, 28040 Madrid (ES)
(72) Inventor: ORELLANA MORALEDA, Guillermo, E-28040 Madrid (ES); LÓPEZ RODAS, Victoria, E-28040 Madrid (ES); COSTAS COSTAS, Eduardo, E-28040 Madrid (ES); HAIG FLOREZ, David, E-28023 Aravaca (Madrid) (ES); MANEIRO PAMPÍN, Emilia, E-28400 Collado Villalba (Madrid) (ES)
(74) Representative: Carpintero Lopez, Francisco
(86) International application number: PCT/ES2008/000465
(87) International publication number: WO 2009/013370

(57) **Abstract**

The present invention provides biosensors based on microalgae for the determination of the presence of toxic compounds in a sample, **characterized by** the high sensitivity and specificity thereof with respect to the target toxic substance. The biosensors are based on the measurement of the photosynthetic activity of algae by means of monitoring the molecular oxygen production by the microalgae by using a luminescent compound the emission of which depends on the amount of oxygen in the medium.

## Description

### Technical Field of the Invention

The invention relates to the field of biosensors based on microalgae and, more specifically, to biosensors for the detection of environmental pollutants by means of the specific monitoring of the inhibition of the photosynthetic activity of the microalgae and the consequent inhibition of the molecular oxygen production by the microalgae in the presence of the toxic compound.

### Background of the Invention

The anthropogenic pollution of ecosystems is currently a hot button issue, not only because of the risk of decreasing the quality and the amount of natural resources, but because said pollution can affect human health. Water is a limited natural resource and the studies conducted both in natural (lakes, rivers,...) and artificial (reservoirs) aquatic ecosystems inform about the increase of the appearance of new toxic agents from industrial, agricultural and domestic activities. There is therefore an increasing demand for improving the systems used to control water pollution by developing fast and reliable devices allowing the detection *in situ* and in real time of the toxic agent, in addition to its precise identification (herbicide, fungicide, insecticide, heavy metals,...) and quantification (see, for example Buffle and Horvai (Eds.), "In Situ Monitoring of Aquatic Systems: Chemical Analysis and Speciation", IUPAC Series on Analytical and Physical Chemistry of Environmental Systems Vol. 6, Wiley, Chichester, UK, 2000; Ligler and Taitt, "Optical Biosensors: Present and Future", Elsevier Science, New York, 2002; Knopf and Bassi (Eds.), "Smart Biosensor Technology", CRC Press, Boca Ratón, Florida, USA, 2006).

Among other systems for detecting toxic agents, chemical biosensors based on non-genetically modified live cells (cells of mammals, bacteria, microalgae,...), parts of cells (membranes, cell organelles), whole organisms (brine shrimp, Daphnia) or enzymatic reactions (oxidases, luciferase, alkaline phosphatase,....) have been proposed as suitable candidates for the detection of water pollution, although few of them have achieved a broad commercial diffusion up until now (Kissinger, 2005, Biosens. Bioelectron., 20, 2512-2516).

Biosensors based on whole cells can be an attractive option provided that the microorganisms which are used as elements of recognition of the analyte are easy to isolate and manipulate, are innocuous, their culture and maintenance is inexpensive, they withstand the necessary handling to integrate them in the biosensor and provide reliable and specific information about the presence and concentration of the target toxic agent. The essence of a biosensor of whole cells is that its cell activity is sufficiently sensitive to the presence of the pollutant of the medium in which the device is introduced, but is hardly affected or is insensitive to the physicochemical characteristics of said medium, to the cell cycle and to the availability of nutrients. Likewise, it must be possible to integrate it easily in the suitable (optical, electrical) transducer of the messenger or biological signal.

Green microalgae are the main components of the population of phytoplankton. Therefore, they can be found in virtually any aquatic medium of the planet. Some species of green microalgae can grow in almost any environmental condition and survive at low concentrations of nutrients or low environmental conditions which are lethal for many other microorganisms (temperature, pH, salts, etc.). It is therefore not surprising that microalgae have already been used to develop biosensors capable of responding to critical changes in aquatic ecosystems (Merz, D. et al. 1996, Fresenius J. Anal. Chem., 354, 299-305; Frense, D. et al., 1998, Sensors Actuators B: Chem., 51, 256-260; Naessens et al., 2000, Ecotoxicol. Environ. Safety, 46, 181-185; Védrine et al. Biosens. Bioelectron. 18, 457-463).

Said biosensors are based on the inhibitory effect of some toxic substances on the photosynthetic activity of algae. The status of the photosynthetic activity of green algae can be monitored by the fluorescence intensity emitted by their chlorophyll molecules or by the molecular oxygen (O₂) production. This is due to the fact that sunlight is absorbed by a network of "antenna" pigments bound to proteins and processed as a result of the photosynthetic reaction center ("photosystems I and II"). A small part of the energy absorbed in excess is dissipated in the form of heat, whereas another part of the absorbed energy is emitted as fluorescence from chlorophyll (Maxwell and Johnson, J. Exper. Botany 2000, 51, 659-668). Sunlight is mainly used to carry out the photolysis of water, with O₂ generation. In the presence of toxic agents, the chlorophyll function is altered, such that the fluorescence is attenuated and the O₂ production decreases. The photosynthetic activity likewise causes an alkalization of the culture medium which is inhibited in the presence of toxic agents.

Microalgal biosensors are thus known in the state of the art in which the photosynthetic function is detected by means of measuring the attenuation of the fluorescence, by means of the decrease of the O₂ production or by means of the inhibition of the alkalization of the medium.

Algae biosensors based on the detection of the fluorescence associated to chlorophyll are mostly based on the measurement of the fluorescence emission of chlorophyll a (*Chl a*) in photosystem II (PSII). This type of biosensor is used because it allows detecting certain herbicides inhibiting electron transport (which are 50% of the herbicides used in agriculture) during the photosynthesis at the level of photosystem II (PSII). This type of biosensor has been extensively described in the literature. Thus, Nguyen-Ngoc and Tran-Minh (Anal. Chim. Acta, 2007, 583, 161) have described a biosensor which allows detecting the pesticide diuron in water by using plant cells (*Chlorella vulgaris*) immobilized in an inorganic translucent support produced by means of sol-gel technology. A similar biosensor for analyzing pesticides in water, but immobilizing said plant cells on a glass filtration membrane, has been described by Naessens et al. (Ecotoxicol. Environ. Saf. 2000, 46, 181-185 and Vedrine. C. et al. (Biosensors and Bioelectronics 2003, 18:457-463)). Altamirano et al. (Biosens. Bioelectron. 2004, 19, 1319-1323) have described a selective biosensor for detecting trinitrotoluene (TNT) by using a wild type and another resistant type of *Dictyosphaerium chlorelloides.* The measurement is based on the inhibition of the fluorescence of photosystem II after 3 minutes of exposure to 0.5-31.3 mg/L of TNT in water. Other examples of biosensors based on the measurement of the intrinsic fluorescence of chlorophyll include the biosensors described by Conrad et al., (J. Appl. Phycol. 1993, 5:505-516) Rizzuto et al. (in F. Mazzei and R. Pilloton (Eds.), Proceedings of the Second Workshop on Chemical Sensors and Biosensors, E.N.E.A., Rome, Italy, 2000); López-Rodas et al., (Eur. J. Phycol. 2001, 36, 179-190); Costas et al., (Eur. J. Phycol. 2001, 36, 179-190), Podola, B. et al., 2004, Biosensors and Bioelectronics, 19:12531260, , Rodríguez, M. et al., (Biosensors and Bioelectronics, 2002, 17:843-849), Sanders, C.A. et al. (Biosensors and Bioelectronics, 2001 , 16:439-446) and Giardi.M.T. et al. (Environ. Sci. Technol., 2005, 39:5378-5384).

These biosensors, however, have the drawback that they only allow detecting those compounds inhibiting PSII, therefore the spectrum of analytes which can be identified is highly reduced. These biosensors further have the added drawback that the fluorescent signal which must be captured is subject to considerable variations not only with the concentration of the target analyte (pesticide, explosive, etc.), but also with the fluctuations in the population of immobilized plant cells, without it being possible to correct this interference.

These problems can be solved by means of using biosensors based on the inhibition of oxygen release, since they allow detecting not only the compounds acting on PSII but any other compound compromising the viability of the microalgae and since the detection of oxygen is subject to less interferences than the detection of the intrinsic fluorescence of microalgae.

The measurements of the generated O₂ can be carried out both optically and electrochemically (Gula, R. N. et al., "Electrochemical and Optical Oxygen Microsensors for In Situ Measurements", in In Situ Monitoring of Aquatic Systems: Chemical Analysis and Speciation, Buffle and Horvai (Eds.), Wiley, New York, 2000). Most of the microalgal microsensors described to date are amperometric devices, which measure the evolution of the photosynthetic O₂ produced in the presence of the toxic substance (for example atrazine or other water pollutants). Examples of such biosensors are those described by Shitanda et al. (Analytica Chimica Acta 2005, 530, 191-197), Pandard et al. (Water Research 1993, 27, 427-431), Campanella, L et al. (Water Res., 2000, 35:69-76). This type of biosensor has the drawbacks of (i) its large size, (ii) the need for a frequent maintenance, (iii) its sensitivity to the contamination by certain chemical species present in water (for example sulfide and (iv) its relatively low sensitivity.

Biosensors in which a measurement of the intrinsic fluorescence of chlorophyll and of the O₂ production is carried out simultaneously by means of amperometry have been described in DE4332163.

Orellana, G and Moreno-Bondi, M.C et al. (Proceedings of the Symposium on Photonics Technologies for 7th Framework Program, pp. 291-294) have described optical sensors for the detection of oxygen based on the capacity of this molecule to deactivate the luminescent emission of certain coordination compounds of Ru(II). However, these sensors have only been used for the determination of the biochemical oxygen demand (BOD) by means of incorporating thereto bacteria which are capable of consuming O₂ in the presence of said organic matter.

Regardless of the previously indicated drawbacks, the analytical capacity of known biosensors is limited by the low specificity to the target analyte, given that they are capable of detecting any substance inhibiting photosynthesis (Koblizek, M. et al. Biotechnol. Bioeng. 1998, 60, 664-669; Merz, D. et al. Fresenius J. Anal. Chem. 1996, 354, 299- 305; Frense, D. et al. Sensors Actuators B: Chem. 1998, 51 , 256-260). To date, the only attempt to obtain a microalgal biosensor specific for a certain analyte is the example of Altamirano, M. (above), in which the biosensor contains a microalgae strain which has been selected due to its resistance to the target analyte which is to be detected and a strain which is sensitive to the target analyte, such that in the presence of the target analyte only the sensitive strain shows a decrease of the photosynthetic activity, whereas in the presence of any other toxic agent, a decrease of the photosynthetic activity of the two microalgae strains is observed. However, this type of study was not implemented in the form of biosensor and, furthermore, it was based on the detection of the intrinsic fluorescence of PSII with the problems involved in this determination as has been discussed above.

There is therefore a need in the art for biosensors which allow specifically detecting toxic substances but which do not have all the problems associated to the determination of the photosynthetic activity of algae by means of quantifying the intrinsic fluorescence associated to chlorophyll.

### Summary of the Invention

In a first aspect, the invention relates to a specific biosensor for detecting a toxic compound comprising
a) a first population of algae the oxygen production of which decreases in the presence of said compound,
b) a second population of algae the oxygen production of which does not vary substantially in the presence of said compound, of the same species as the first population and which has been obtained by means of a process comprising the steps of
   (i) a first selection of spontaneous mutants viable to said compound by means of exposure to said toxic compound and
   (ii) a second selection from the mutants obtained in step (i) in the presence of progressively higher concentrations of said toxic compound by means of at least one ratchet cycle.
c) means for stimulating the photosynthetic activity of the two populations of algae and
d) means for detecting the oxygen produced by each of the two populations of algae **characterized in that** the means for detecting the oxygen produced by each of the populations of algae comprise at least one compound the optical properties of which vary according to the concentration of oxygen in the medium and at least one optoelectric element suitable for detecting the optical properties of said compound.

In a second aspect, the invention relates to the use of a biosensor of the invention for detecting the presence of a toxic compound in a sample.

In a third aspect, the invention relates to the use of a biosensor of the invention for distinguishing the presence of the toxic compound causing a decrease of the oxygen production in the first population of algae from other toxic compounds in a sample.

In a fourth aspect, the invention relates to a method for the detection of a toxic compound in a sample comprising the steps of
a) contacting the sample with a biosensor according to the invention wherein the second population of algae has been selected in the presence of said toxic compound and
b) detecting the production of light of each of the populations of cells in the biosensor in the presence of said sample
wherein a decrease in the production of light of the first population of cells with respect to the second population of cells indicates that the sample contains the toxic compound against which the cells of the second population have been selected and wherein a decrease in the production of light by the two populations of cells indicates that the sample contains a toxic compound different from the compound against which the first population of cells has been selected.

In a fifth aspect, the invention relates to a process for obtaining microalgae resistant to a toxic compound comprising the steps of
a) a first selection of spontaneous mutants viable in the presence of said toxic compound and
b) a second selection from the mutants isolated in step (a) in the presence of progressively higher concentrations of said toxic compound by means of at least one ratchet cycle.

In a sixth aspect, the invention relates to an algae strain resistant to a toxic compound which can be obtained by means of the selection process of the invention.

### Brief Description of the Drawings

Figure 1 shows a graphic depiction of the steps to be followed in Set 1 and Set 2.
Figure 2 shows an experimental diagram of the ratchet cycles in which in a first cycle the microalgae are grown in the presence of 1/10 of the LD100, 1/3 of the LD100 and the LD100 of the toxic agent. If the microalgae grow at all the concentrations, the following cycle is performed, wherein the microalgae are grown in the presence of 1/3 of the LD100.
Figure 3 shows a diagram of the result of the selection process by means of ratchet cycles with the different strains which showed a higher resistance to the toxic agent.
Figure 4 shows the characterization of the oxygen production during the photosynthesis of the strain which is most sensitive to the toxic agent.
Figure 5 shows the oxygen production during the photosynthesis of the strain which is most sensitive to the toxic agent.
Figure 6 shows the selection of the concentration inducing a 100% lethal dose.
Figure 7 shows the oxygen production of resistant microalgae in the absence or presence of toxic agent.
Figure 8 shows the oxygen production of sensitive and resistant microalgae in the absence or presence of toxic agent.
Figure 9 shows the oxygen production of sensitive and resistant microalgae in the presence of the target toxic agent.
Figure 10 shows the colonization of the microporous matrix by the resistant microalgae. Left panel: Non-colonized ImmobaSil membrane. Top right panel: ImmobaSil membrane colonized by 5x10⁵ cells. Bottom right panel: Cross-section of the ImmobaSil membrane colonized by the microalgae.
Figure 11 shows a diagram of the biosensor wherein S and R indicate the heads containing the microalgae strains sensitive (S) and resistant (R) to the target analyte.

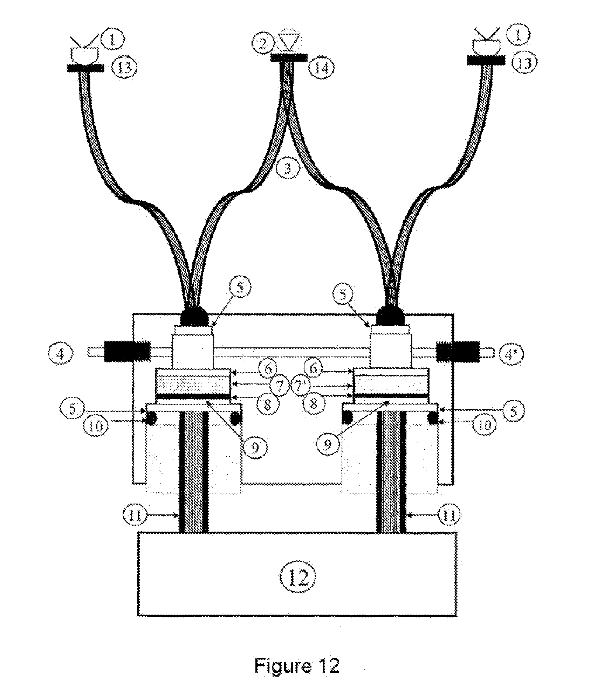

Figure 12 shows a diagram of the biosensor which indicate the blue LED (470 nm) powered by a current source (1), the visible photodiode or CCD sensor (2), the optical glass trifurcated optical fiber (3), the device for the entrance (or exit) of the sample (4), the device for the exit (or entrance) of the sample (4'), the plastic or optical glass window (5), the cellulose membrane disk (6), the porous silicone disk containing the resistant (or sensitive) microalgae (7), the porous silicone disk containing the sensitive (or resistant) microalgae (7'), the thin layer of material opaque to light (8), the sensor membrane permeable to oxygen (9), the O-ring for sealing the measurement cavity (10), the bifurcated optical fiber (11), the measurement optoelectronic unit with luminescent sensors (12), the blue band-pass optical filter (13) and the long-pass or cutoff optical filter (14).
Figure 13 shows a detailed diagram of the head (the references correspond to those of Figure 12).

### Detailed Description of the Invention

The present invention is based on the unexpected discovery by the authors of the fact that the sensitivity and specificity of the biosensors of toxic products based on algae can be considerably increased if the strains integrating said biosensors have been previously selected by their resistance and/or sensitivity to the toxic agent the presence of which is to be detected, wherein said selection is carried out by means of a first fluctuation analysis step (for the resistant and sensitive strains) and by means of a second selection step using one or more ratchet cycles (in the case of the resistant strains). Without wishing to be bound by any theory, it is believed that the first selection step allows identifying mutants (which either already pre-exist in the culture medium or have appeared spontaneously during the selection) whereas the ratchet cycle or cycles allow selecting organisms accumulating more than one mutation which confer resistance to the toxic compound. Strains showing an increased sensitivity to the target toxic substance (providing higher sensitivity to the target toxic substance) and strains showing an increased resistant to the target toxic substance (providing higher specificity) are thus obtained.

The inventors have likewise observed that it is possible to increase the versatility of the biosensors by means of the direct detection of the molecular oxygen instead of the measurement of the fluorescence associated to chlorophyll, and that said direct detection of oxygen can be carried out using the same type of sensor which is used for the detection of BOD, in which the oxygen is not measured amperometrically but by means of using a luminescent compound the luminescence of which is partially deactivated in the presence of O₂, such that a decrease in the O₂ production by the microalgae as a result of the decrease of the photosynthetic activity results in an increase of the luminescence by said compound.

Thus, in a first aspect, the invention relates to a biosensor based on microalgae which is specific for a certain toxic compound, wherein said biosensor comprises the following elements:
a) a first population of algae the oxygen production of which decreases in the presence of said compound,
b) a second population of algae of the same species as the first population, the oxygen production of which is not modified in the presence of said compound and which has been obtained from a wild-type algae strain by means of a process comprising the steps of
   (i) a first selection of resistant mutants in the presence of said toxic compound and
   (ii) a second selection from the mutants obtained in step (i) in the presence of progressively higher concentrations of said toxic compound by means of at least one ratchet cycle,
c) means for stimulating the photosynthetic activity of the two populations of algae and
d) means for detecting the oxygen produced by each of the two populations of algae **characterized in that** the means for detecting the oxygen produced by each of the populations of algae comprise at least one compound the optical properties of which vary according to the concentration of oxygen in the medium and at least one optoelectronic element suitable for detecting the optical properties of said compound.

As used in the present invention, biosensor is understood as a device capable of carrying out analytical determinations (typically *in situ* and in real time) which contains, physically linked or confined, a biological element of recognition in direct contact with the sample and spatially close to or in contact with said transducer element, regardless of the transducer element providing a measurable signal (optical, electrochemical, piezoelectric, mass signal,...).

In the context of the present invention, the biosensor comprises two populations of microalgae which have been selected for their resistance and/or sensitivity to a certain analyte. As used in the context of the present invention, microalga is understood as any photosynthetic protest belonging to the *Chlorophyta* type. Illustrative non-limiting examples of algae which can be used in the present invention include microalgae belonging to the genera *Scenedesmus sp., Chlamydomonas sp., Chlorella sp., Spirogyra sp., Dunaliella sp., Euglena sp., Prymnesium sp., Porphyridium sp., Synechoccus sp.* and *Dictyosphaerium sp.* In a preferred embodiment, the alga species forming the first and second population of algae is *Dictyosphaerium chlorelloides.*

There is virtually no limitation with regard to the analytes which can be used to select resistant and sensitive microalgae strains and which therefore can be detected with the biosensor of the invention as long as said compounds have a toxic effect against the microalga and produce an inhibition of the photosynthesis. Illustrative non-limiting examples of these types of compounds include herbicides (for example simazine, DCMU, glyphosate), fungicides (for example 2-phenylphenol, azaconazole, azoxystrobin, carboxin, cymoxanil, cyproconazole, dodine, epoxiconazole, etridiazole, fenfuram, ferimzone, flusilazole, flutriafol, fuberidazole, furalaxyl, furametpyr, imazalil, metalaxyl, methasulfocarb, metominostrobin, myclobutanil, ofurace, oxadixyl, oxycarboxin, phenylmercury acetate, propiconazole, prothioconazole, pyrimethanil, pyroquilon, tetraconazole, thiabendazole, tricyclazole), insecticides (abamectin, acetamiprid, aldicarb, azadirachtin, azamethiphos, bendiocarb, carbaryl, carbofuran, clothianidin, cryolite, dazomet, dimethylvinphos, DNOC, emamectina benzoate, ethiofencarb, ethylene dibromide, fenamiphos, fenobucarb, fipronil, flonicamid, imidacloprid, isoprocarb, lufenuron, methidathion, methyl isothiocyanate, methiocarb, pirimicarb, propoxur, pymetrozine, pyridaphenthion, chlorantraniliprole (Renaxapyr^{™}), sabadilla, spinosad, sulcofuron-sodium, thiacloprid, thiamethoxam, thiofanox, triazamate, XMC and xylylcarb), raticides (chloralose, chlorophacinone, coumatetralyl and strychnine), toxins (for example microcystine LR), antibiotics (for example fluoroquinolones), heavy metals (for example copper, chromium or lead) and the like.

The first population of algae is characterized by being sensitive to the analyte which is to be determined, i.e., the photosynthetic activity of said population decreases or disappears completely in the presence of the analyte. In theory, any microalgae strain sensitive to the analyte can be used in the context of the present invention. However, in a preferred embodiment, the first population has been selected from strains appearing naturally due to their higher sensitivity to the target analyte, i.e., the LD100 of the strain against the analyte is lower than in the original strain. The selection is carried out by means of methods known by the person skilled in the art. In a preferred embodiment, individual clones of algae isolated from nature are grown in the presence of increasing concentrations of the toxic agent and that clone showing a lower cell density in the culture after a certain time is selected. In a preferred form of the invention, the microalgae strains are grown in aliquots of a constant number of cells in the presence of the toxic agent for 5 days.

The second population of cells is characterized by being resistant to the target analyte, i.e., the photosynthetic activity of said population is not substantially modified in the presence of the analyte. The second population of cells is obtained by means of a double selection process comprising a first selection step by means of the fluctuation analysis of mutants which are present in the initial population or which appear spontaneously during the selection of the strains in the presence of the target analyte.

As a starting material for obtaining said second microalgal population, the invention requires using algae of the same species as the strain which is used as the first population. However, in a preferred embodiment, the microalgal strain which is used as the starting material in the first selection cycle is the strain of the first population characterized by being especially sensitive to the target analyte.

The first selection step is carried out by means of culturing clones isolated from the alga which is to be used in the presence of increasing concentrations of the target analyte and selecting those strains having a higher cell density after a certain culture period. The selection can be carried out using as a starting material cultures with a low density or with a high cell density or using both types of cultures simultaneously. In a preferred form of the invention, cultures with a high and with a low cell density are used simultaneously, such that the statistical analysis of the number of resistant strains allows deducing if the mutant existed originally in the microalgal population before the exposure to the toxic agent or, on the contrary, if the mutation is the result of a physiological adaptation induced by the continued exposure to the toxic agent.

The second selection step is carried out by means of the so-called ratchet cycles, using to that end methods known by the person skilled in the art. In a preferred embodiment, the ratchet cycles are carried out by means of the method described by Reboud, X. et al. (Biological Journal of the Linnean Society, 2007, 91 :257266). In an even more preferred embodiment, the starting point is several cultures of the strain or the starting clone to which concentrations of toxic agent equal to or greater than the LD100 are added and they are maintained for a certain time (first ratchet cycle). If it is observed that there is algal growth after said period, the concentration of toxic agent is increased and the algae are maintained in culture for the same time (second ratchet cycle). The cycles are repeated as many times as necessary until any replicate of the resistant clone does not grow, which preferably requires in the order of 10 to 15 cycles, depending on the type of chemical agent. At the time, the optimization is considered ended.

The person skilled in the art will understand that the strains sensitive and resistant to the target toxic substance can be identified by means of standard techniques known for the person skilled in the art. Thus, the resistant strains can be identified by means of determining the LD100 of the particular strain for said target toxic substance, such that if the LD100 is above the LD100 value for the starting strain, the strain would be a strain which has been obtained by the method of the invention. Likewise, the sensitive strains could be identified by means of determining the LD100, such that if said LD100 is less than the LD100 of the starting strain, the strain would be a strain which could be incorporated in the biosensor of the invention.

The means for stimulating the photosynthetic activity of the populations of algae comprise a light source with a wavelength suitable for stimulating the photosynthesis of the populations of microalgae. To that end, any light source producing light with a peak wavelength at about 470 nm can be used in the context of the present invention. The invention contemplates the use of sources of multichromatic or monochromatic light. In the case of panchromatic sources, the light can be used as such or broken down into elemental beams of light by means of standard monochromators. However, the use of monochromatic light sources such as lasers and LEDs is preferred. In a preferred embodiment, the light source is a blue LED emitting a narrow band centered at 470 nm.

The optoelectronic element (12) which serves to detect the optical properties of the compound the properties of which vary in the presence of the compound to be detected includes an element exciting the photoluminescence of the film sensitive to oxygen, continuously measures the intensity and/or phase difference thereof, correlates these parameters with the concentration of the toxic substance in the water and controls the start, duration and intensity of the light pulse of the source. The luminescence emitted by any detector of those known in the art, including linear diode arrays, charge-coupled devices (CCD) or any other device sensitive to light. The optoelectronic unit can also control the actuation of auxiliary pumps causing the flow of the water sample through the sensitive end.

Compounds the luminescent properties of which vary in the presence of O₂ include complexes of transition metals, particularly, platinum d₆ metals such as Ru(II), Os(II), Re(II), Pt(II), Rd(lll) and Ir(III) with at least one diimine-type ligand (for example, 2,2'-bipyridine, 1,10-phenanthroline and derivatives thereof with different degree of substitution). Conjugated phenyl rings can preferably be introduced in the previously defined structures for the purpose of increasing the sensitivity to oxygen. Illustrative non-limiting examples of luminescent complexes which can be used in the context of the present invention include tris(1,10-phenanthroline)ruthenium(II), tris(4,7-diphenyl-1,10-phenanthroline)ruthenium(II), tris(2,2-bipyridine)ruthenium(II), tris(4-phenyl-7-para-hexanolphenyl-1,10-phenanthroline)ruthenium(II) (5-isothiocyanate-1,10-phenanthroline)bis(2,2-bipyridine)ruthenium(II), as well as all the luminescent compounds described in WO/1998/053316.

In another preferred embodiment, the compound the optical properties of which vary in the presence of oxygen is impregnated in a film permeable to O₂ (9). This type of membrane is known to the person skilled in the art (for example the membranes described by Navarro-Villoslada et al. (Anal. Chem, 2001, 73:5150-5156) which are preferably obtained by means of copolymerizing substituted methacrylate units with phosphorylcholine and dodecyl methacrylate. The sensor film preferably has a thickness of between 5 to 250 µm. In another embodiment, the film sensitive to oxygen is separated from the microalgae by a thin layer of an opaque polymer permeable to oxygen (8), such that the oxygen released by more microalgae can access the sensor film but the light from the diode stimulating the photosynthesis of the populations of algae (1) cannot. In general, any membrane permeable to oxygen can be used in the context of the present invention to coat the sensor film (8), but for the use in the present invention, membranes made of silicone, of polydimethylsiloxanes or of cellulose acetate are preferably used.

In another embodiment, the two populations of algae are immobilized in a support. In a preferred embodiment, the support is a three-dimensional matrix. In an even more preferred embodiment, the three-dimensional matrix is a microporous matrix. Virtually any microporous support can be used in the present invention, provided that it is suitable for culturing cells in suspension and that the pore size is small enough to prevent the algae from escaping from its inside. Suitable materials for preparing microporous supports according to the present invention include gelatin (Cultispher G, HyClone), cellulose (Cytocell, Pharmacia), polyethylene (Cytoline 1 and 2, Pharmacia), silicone (Immobasil, Ashby Scientific), collagen (Microsphere, Cellex Biosciences), crystal (Siran, Schott Glassware), acrylic copolymers, copolymers of acrylamide, methylene-bisacrylamide, dimethylaminopropyl, methacrylamide, methylstyrene methacrylate, ethylene/acrylic acid, acrylonitrile butadiene styrene (ABS), ABS/polycarbonate, ABS/polysulfone, ABS/polyvinyl chloride, ethylene propylene, ethyl vinyl acetate (EVA), nitrocellulose, polyacrylonitrile (PAN), polyacrylate, polycarbonate, polybutylene terephthalate (PBT), polyethylene terephthalate (PET), polymers and copolymers of polypropylene, polystyrene, polytetrafluoroethylene (PTFE), fluorinated ethylene-propylene (FEP), ethylene tetrafluoroethylene (ETFE), perfluoroalkoxyethylene (PFA), polyvinyl fluoride (PVF), polyvinylidene fluoride (PVDF), polychlorotrifluoroethylene (PCTFE), ethylene chlorotrifluoroethylene (ECTFE), polyvinyl alcohol (PVA), styrene acrylonitrile (SAN), styrene-maleic anhydride (SMA).

In another embodiment, the microalgae are separated from the aqueous medium in which the measurement is carried out by means of a membrane impermeable to the microalgae. The loss of the microalgae by washing is prevented and at the same time the membrane containing the microalgae is protected from becoming dirty and from contamination. Suitable materials for the impermeable membrane include cellulose, paper and cellulose derivatives (cellulose acetate and cellulose nitrate), polyester, cellulose nitrate, polycarbonate, microporous polyethersulfone, PET, polyvinylidene fluoride (PVDF), polypropylene, silica, alumina, diatomaceous earth, gelatin, polyacrylamide and the like. These materials can be used in the membrane independently or in combinations of one or several and can be subjected to treatment for the purpose of increasing or decreasing their hydrophobicity.

In another embodiment, the biosensor additionally contains means for detecting the fluorescent signal emitted by chlorophyll in response to the light which is used to stimulate photosynthesis. The fluorescent emission by each population of algae, when it is measured in the absence of toxic agent, is directly proportional to the population of microalgae, which allows correcting in real time the signal from the oxygen sensors and increasing the precision, accuracy and time stability of the measurement of the analyte. The detection of the fluorescent signal is carried out by means of a sensor (2) sensitive to red light, preferably a visible photodiode or a CCD sensor. The fluorescent signal is preferably transmitted from the microporous membrane (7 or 7') to the detector (2) by means of an optical fiber bundle (3). In a preferred embodiment, the optical fiber bundle transmitting the actinic light from the light source (1) and the optical fiber bundle transmitting the fluorescence signal from the immobilized algae to the sensor (2) form part of a single trifurcated optical fiber (3).

The biosensor which is the object of the present invention is to date the only available biosensor based on microalgae in which the selection has been unified without the genetic manipulation of strains that are highly sensitive to the toxic agent, strains resistant to the toxic agent and the immobilization in a porous three-dimensional membrane of both to be jointly used for the purpose achieving the maximum sensitivity and specificity with respect to a toxic agent. The combination of all these characteristics included in a biosensor the signal of which is the integration of both strains (sensitive and resistant) has the great advantage that it does not involve any risk when using the *in situ* biosensor for measurements in aquatic ecosystems, since the biological component is from the same aquatic environment and the latter is not genetically manipulated.

In another aspect, the invention relates to the use of the biosensor of the invention for detecting the presence of a toxic compound in a sample.

In another aspect, the invention relates to the use of the biosensor of the invention for distinguishing in a sample the presence of the toxic compound against which the second population of cells has been selected from other toxic compounds.

The samples which can be analyzed by using the biosensor of the invention include any conventional sample in which it is suspected that a toxic compound can appear, such as for example, river water, water from a reservoir, water from a treatment plant, water from an industrial effluent and the like. In the event that the sample suspected of containing a toxic agent is solid (for example, food), it is necessary to perform an extraction of molecules soluble in aqueous solvents, such that said extract can be applied to the biosensor of the invention. The person skilled in the art will understand that the use according to the present invention is not limited to studying samples which are potentially contaminated with the target toxic substances, but can be used at industrial level in production plants of said toxic agents to monitor the yield and purity of the synthesis.

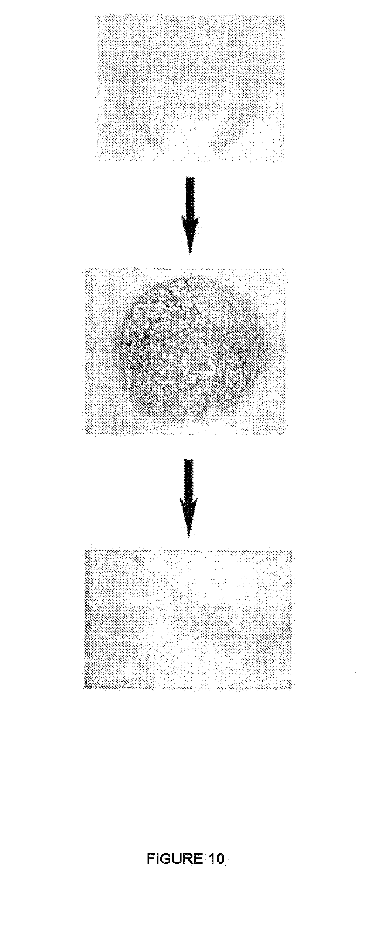

In use, the biosensor works as follows. The water sample is applied through the conduit (4) allowing the access of the sample to the heads containing the sensitive and resistant algae (Figure 10). Each head comprises a cellulose membrane disk (6) associated to the porous membrane (7 or 7') in which the cells are located and which is in turn in contact with an opaque porous membrane (8) allowing the passage of oxygen to the oxygen sensor membrane (9). The entire measurement cavity is hermetically sealed by means of respective O-rings (10) on both sides of the porous membrane (7 or 7'). For the illumination of the heads, the actinic radiation emitted by the blue LEDs (1) filtered by means of blue band-pass optical filters (13) and optical fiber bindles directing the illumination from the LED (1) to a glass window (5) which is contact with the membrane disk (6) of the head are used. The illumination excites the photosynthetic activity of the cells which are located in the porous membrane (7) which produce oxygen, which diffuses through the opaque porous membrane (8) until reaching the sensor membrane (9) wherein they shield the luminescence of the compound impregnating said sensor membrane (9). In the presence of the toxic compound which has been used to select the resistant cells, the photosynthetic activity of the sensitive cells which are located in the first head (7) decreases, whereas the photosynthetic activity of the resistant cells which are located in the second head (7') remains unchanged. The decrease of the photosynthetic activity results in a decrease of the oxygen production by the sensitive cells which in turn results in a decrease of the deactivation of the luminescent signal, whereby there is an increase of the luminescence of the sensor membrane. The luminescent signal is transmitted from the heads by means of bifurcated optical fiber bundles (11 or 11') to the measurement optoelectronic unit (12). In the presence of an unspecific toxic compound, there is a decrease of the photosynthetic activity by both heads, which results in an increase of the luminescent signal by the two heads. For the purpose of simultaneously determining the amount of algae in each of the porous membranes (7 or 7'), the fluorescent emission associated to chlorophyll in the absence of toxic agent is determined, using to that end a photodiode or CCD sensor (2) to which the fluorescent emission of the population of algae is sent by means of an optical fiber bundle which together with optical fiber used to transmit the excitation radiation from the blue LED (1) forms a trifurcated optical fiber (3). Before reaching the sensor (2), the fluorescent emission is passed through a cutoff optical filter (14). The simultaneous measurement of the fluorescence of the two populations of algae is a measure of the amount of immobilized biomass, which allows correcting in real time the signal from the oxygen sensors, simultaneously the amount of immobilized biomass and thus standardizing the luminescent emission to the amount of algae.

Thus, in another aspect, the invention relates to a method for the detection of a toxic compound in a sample comprising the steps of
a) contacting the sample with a biosensor of the invention wherein the second population of algae has been selected in the presence of said toxic compound and
b) detecting the production of light of each of the populations of cells in the biosensor in the presence of said sample
wherein a decrease in the production of light of the first population of cells with respect to the second population of cells indicates that the sample contains the toxic compound against which the cells of the second population have been selected and wherein a decrease in the production of light by the two populations of cells indicates that the sample contains a toxic compound different from the compound against which the first population of cells has been selected.

In the method of the invention, the luminescence values of each of the cell populations depend not only on the inhibition of the photosynthetic activity in each of the populations but also of the amount of immobilized cells in each population. To that end, in a preferred embodiment, the values of production of light by each of the cell populations are standardized against the total amount of cells in each population. The standardization requires the simultaneous determination of the total amount of algal biomass in each immobilized population and the standardization of the values of variation in the production of light by the two populations of algae according to the amount of immobilized algal biomass. In an even more preferred embodiment, the determination of the amount of immobilized algal biomass is carried out by means of detecting the fluorescent emission of each population of algae in response to the stimulation of the photosynthetic activity in the absence of toxic substance. Preferably, the determination of the biomass of each of the algal populations is determined simultaneously to the determination of the luminescent signal over time, which allows correcting the values in real time. In an even more preferred embodiment of the invention, the optoelectronic unit (12) is responsible for carrying out the standardization based on the values of luminescence emitted by the sensor membrane (9) and of the intensity of the fluorescent signal detected by the sensor (2).

The authors of the present invention have shown that the specificity of the biosensor has only been possible as a result of the incorporation in the sensor of cells that are highly resistant to the target toxic substance. Said cells have been obtained in an inventive manner by the authors of the invention by means of the surprising observation of the fact that the application of a selection by ratchet cycles to cells which already showed resistance to the target toxic substance allows obtaining cells with an even higher resistance than the starting cells. Without wishing to be bound by any theory, it is believed that the increased resistance of the cells obtained by the method of the invention is based on the capacity of the ratchet cycles to select mutants containing multiple mutations capable of conferring resistance to the toxic agent.

Additional advantages of the selection method of the invention include
- It is possible to obtain microalgae sensitive and resistant to herbicides, fungicides, insecticides, raticides, metals,... etc.
- The manipulation to obtain sensitive and resistant microalgae is carried out without needing to alter the genetic load of the microalgae, therefore there is no risk of accidental contamination in the aquatic medium of microalgae different from those already existing in it.
- The integration in the biosensor of resistant microalgae confers specificity thereto, i.e., type of toxic agent, the bioavailability thereof and the concentration thereof in the aquatic medium.

Thus, in another aspect the invention provides a process for obtaining microalgae resistant to a toxic compound comprising the steps of
a) a first selection of viable spontaneous mutants in the presence of said toxic compound and
b) a second selection in the presence of progressively higher concentrations of said toxic compound from the cells obtained in step (a) by means of at least one ratchet cycle.

There is no limitation with regard to the suitable algae species as well as to the suitable toxic compounds for carrying out the selection method. However, the preferred species and the preferred compounds have been set forth above. In a preferred embodiment, the toxic compound used in selection steps (a) and (b) is simazine. In another preferred embodiment, the alga which is subjected to selection is *Dictyosphaerium chlorelloides.* Step (b) preferably includes at least 2 ratchet cycles, more preferably at least three ratchet cycles.

The selection method of the invention allows obtaining a population with a degree of resistance to the target toxic agent much higher than the use of each of the methods separately. Without wishing to be bound by any theory, it is believed that this technical effect is a consequence of the use as a starting material of cells which have already been selected for their resistance to the toxic compound. If the selection were performed only by means of step (a) or by means of step (b), the resulting cell population would contain a mixture of sensitive and resistant strains, which would make them unacceptable for their incorporation to a biosensor such as the one which is the object of the invention.

In another aspect, the invention relates to algae resistant to a certain toxic substance which have been obtained by means of the selection method of the present invention.

The invention is detailed below in an illustrative manner with the following examples which must be interpreted in an illustrative and non-limiting manner.

### Examples

### Example 1

### Obtaining sensitive and resistant microalgae

Sterile commercial material was used for the culture of microalgae, which comprise sterile 96-well plates, sterile 4-well plates, 25 cm² culture flasks, sterile plastic pipettes, sterile BG-11 culture medium, laminar flow cabinet, culture chamber for microalgae, inverted microscope, optical microscope, fridge at 4ºC, stereomicroscope, oximeter, Neubauer chamber.

A water sample was collected in a sterile flask in the area in which the biosensor will be located. The microalgae for obtaining strains are cloned by isolating the latter (for example, those belonging to the genus *Dictyosphaerium chlorelloides*) from the sample deposited in a slide and selecting a single cell by means of a micromanipulator, in a laminar flow cabinet. Each isolated cell is transferred to one of the wells of the sterile 96-well plates with BG-11 culture medium (Sigma-Aldrich Química, Tres Cantos, Madrid, Spain). Each individual present in each well after repeated mitoses will give rise to multiple individuals, all of them genetically identical forming a clone. Those strains which showed the followed characteristics were selected:
- Fast growth
- Resistant to the manipulation
- Survival in board environmental variations
- Non-producer of biotoxins

The microalgal cells strains isolated in 96-well plates with BG-11 culture medium are maintained in a culture chamber at 22ºC under continuous photosynthetically active light of 60 µmol/m²/s. After 2 weeks, the content of each well is transferred to sterile 25 cm² flasks with 20 mL of BG-11 medium and kept in a culture chamber under the same temperature and light conditions.

### Example 2

### Selection of the strain which is most sensitive to a certain toxic agent without genetic modification.

The clones which are most sensitive to the exposure of the toxic agent, i.e., the clones significantly decreasing the oxygen production in the shortest time and at the lowest concentration of the toxic agent, were selected from among all the species and strains isolated from the water sample. To that end, exposures of the clones to increasing concentrations (for example 0, 3, 10, 30, 100, 300 µg L⁻¹) of the toxic agent are carried out in triplicate, maintaining the exposure for 5 days. The strain showing a lower density against the concentration of the toxic substance is considered the most sensitive strain. The clones showing the most sensitive and fastest response to the oxygen production were selected from among the clones of least growth. The oxygen production is measured with an oximeter. Aliquots of 5 x10⁵ cells are used, measuring at 1, 2, 5, 10, 15 and 30 minutes.

### Example 3

### Method for the selection of microalgae resistant to the toxic agent without genetic modification by means of fluctuation analysis (fluctuation analysis modified for liquid cultures applied to unicellular microalgae) and optimization of the resistant clones obtained by means of ratchet cycles.

### 3.1 Selection of the clone which is most sensitive to the toxic agent

The clone which is most sensitive to the toxic agent obtained in Example 1 is cultured in the presence of increasing concentrations (for example 0, 3, 10, 30, 100, 300 mg L⁻¹) of toxic agent in BG-11 medium for 5 days. The number of cells in each sample is determined using an automatic counter and that concentration of toxic agent inducing a LD100 lethal dose is selected.

### 3.2 Fluctuation analysis

100 culture tubes (tube volume 5 mL) are then seeded with 100 cells of microalgae of the clone which is most sensitive to the toxic agent at a low cell density and 4 mL of BG-11 medium in each one, called "SET 1 ".

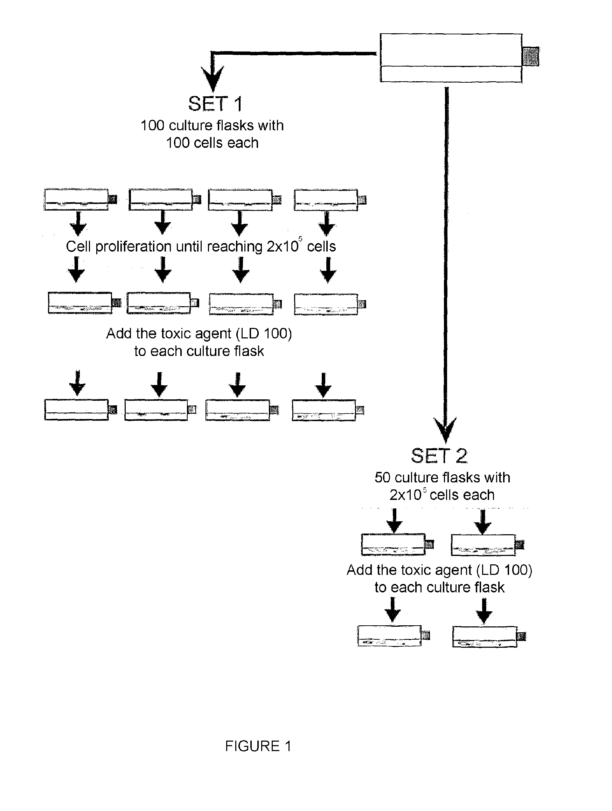

The microalgae in SET 1 are allowed to reach a density of 2x10⁵ microalgae in each of the 100 flasks. The microalgae proliferation is controlled by means of counts in Cell-Coulter. The dose of the toxic agent inducing LD100 is subsequently added to each of the 100 culture tubes and they are left in culture for 1 month. The microalgae in each of the 100 culture tubes are subsequently counted. The number of cells in a tube which was seeded at a cell density of 2x10⁵ microalgae with microalgae of the clone which is most sensitive to the toxic agent with (50 culture tubes with 2x10⁵ microalgae each), called "SET 2" is used as a control. The concentration inducing LD 100 is added to each of the 50 culture tubes and they are left in culture for 1 month and the microalgal count in each of the 50 culture tubes is subsequently carried out (Figure 1).

The statistical processing of the results of Set 1 and Set 2 will indicate if the resistance to the toxic agent is due to a natural mutation which already existed in the microalgal population before its exposure to the toxic agent (pre-selective adaptation or natural resistance) or if, on the contrary, it is a physiological adaptation induced by the continued exposure to the toxic agent (post-selective adaptation or induced resistance). In Set 1, if the resistant microalgae come from a spontaneous mutation before the exposure to the toxic agent, then there should be a high variance in the number of microalgae per tube. If the resistant microalgae come from the exposure to the toxic agent, their presence per tube should be similar and their variance very low. Set 2 serves to estimate the error in the sampling of the resistant cells (López Rodas et al., Eur. J. Phycol. 2001 , 36, 179- 190).

### 3.3 Optimization of the resistant clones by means of ratchet cycles

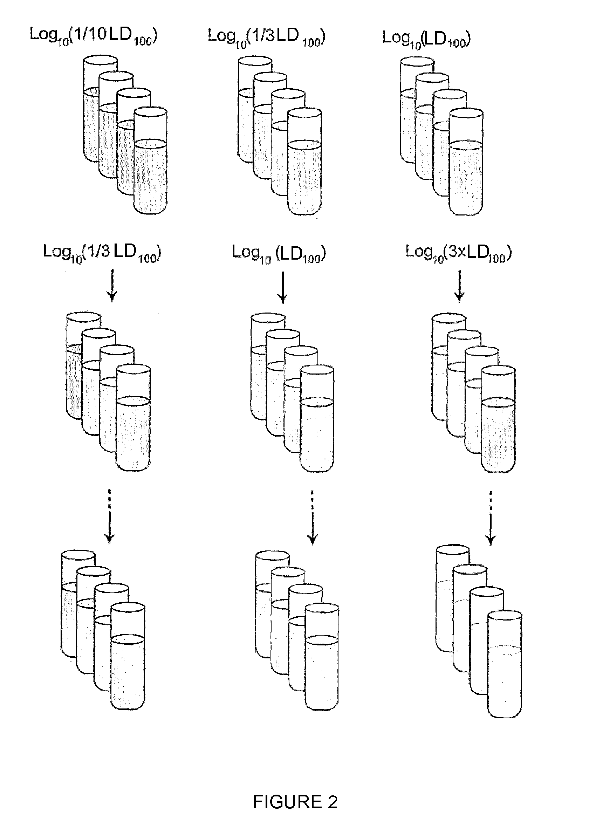

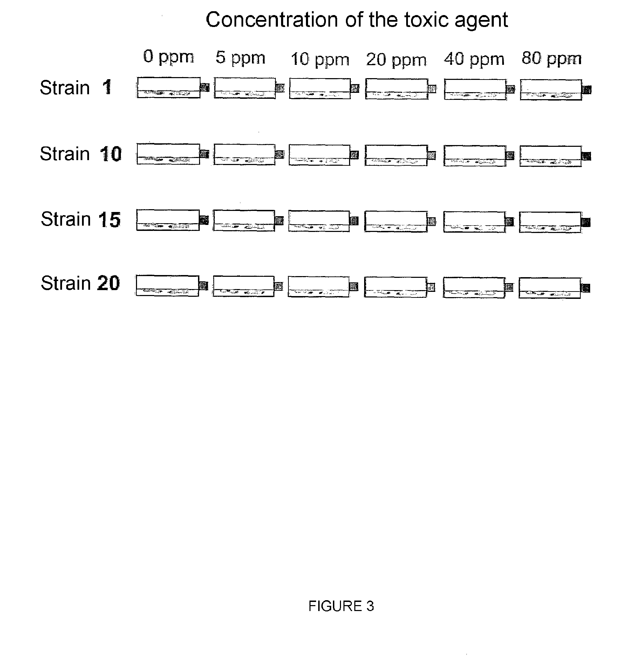

A general diagram of the selection method for selecting resistant strains by means of ratchet is indicated in Figure 2. The resistant clone obtained by means of fluctuation analysis is exposed to the toxic agent by means of ratchet cycles (Reboud et al., 2007, Biological Journal of the Linnean Society, 91, 257-266), which allow the continued exposure of the clone to the toxic substance. The concentration of toxic substance used will be 1/10, 1/3 and 1 of the LD 100. To that end, 3 tubes with 500,000 cells in 4 mL of medium for every concentration used and a control, forming the first ratchet cycle, are taken. If there is microalgal growth in all the tubes after 20 days, the concentration of the toxic agent is increased (double concentration of LD 100), in order to start the second ratchet cycle. This process will be repeated with successive ratchet cycles until the resistant clone does not grow in any replicate (Figure 3). At this time, the optimization is considered ended. Each of the cycles selects the clones with the highest degree of resistance to the toxic agent. It is a method which maximizes the selection.

### Example 4

### Immobilization of microalgae sensitive and resistant to the toxic agent in an ImmobaSil three-dimensional porous membrane (Cellon, Luxembourg).

The Immobasil membranes with a diameter of 10 mm and a thickness of 0.25 m are washed with distilled water, the membranes are sterilized in an autoclave (121ºC, 15 minutes), allowed to dry and stored in a sterile container with distilled water and washed with BG-11 before use. The membranes are then places in sterile 4-well plates, 1 mL of BG-11 medium with 500,000 plant cells of the most sensitive clone per mL in each well are added to each of the wells. The membrane is checked weekly until the microalgae colonize the entire membrane surface (the typical time is 3-4 weeks depending on the type of clone and on the type of microalga used).

### Example 5

### Characterization of the microalgae strain which is most sensitive to the toxic agent. Dose-effect curve.

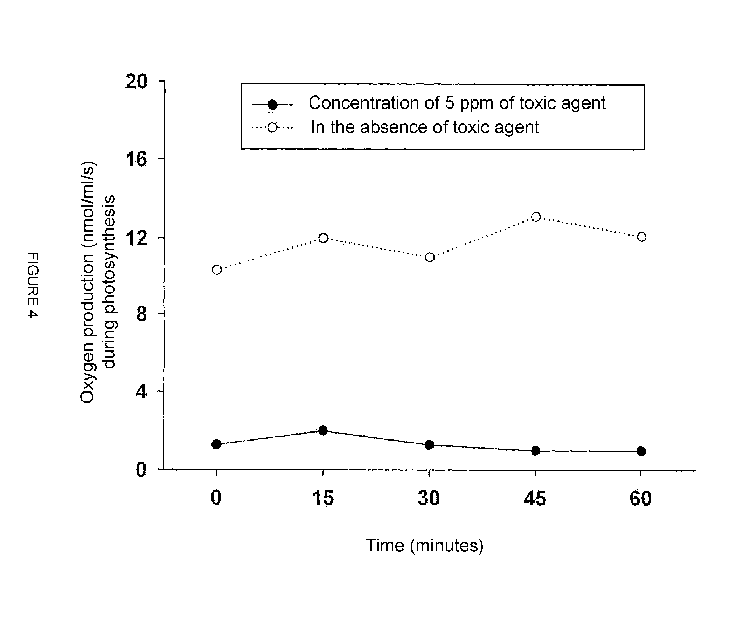

The strain with the best response to the toxic agent from the isolated microalgae strains is strain 20 (see Figure 3). In the same time of response, the highest percentage of inhibition of the oxygen production corresponds to strain 20 and is statistically significant with respect to the rest of the strains. The rate of division of the sensitive microalga is (0.98 ± 0.1), approximately one division every 24 hours in the absence of the toxic agent. As shown in Figure 4, the oxygen production curve of the sensitive microalgae is significantly different in the presence of 5 mg L⁻¹ (ppm) of the toxic agent, from that which is produced in the absence of the toxic agent.

### Example 6

### Characterization of the microalgae resistant to the toxic agent without genetic modification by means of fluctuation analysis modified for liquid cultures applied to unicellular microalgae.

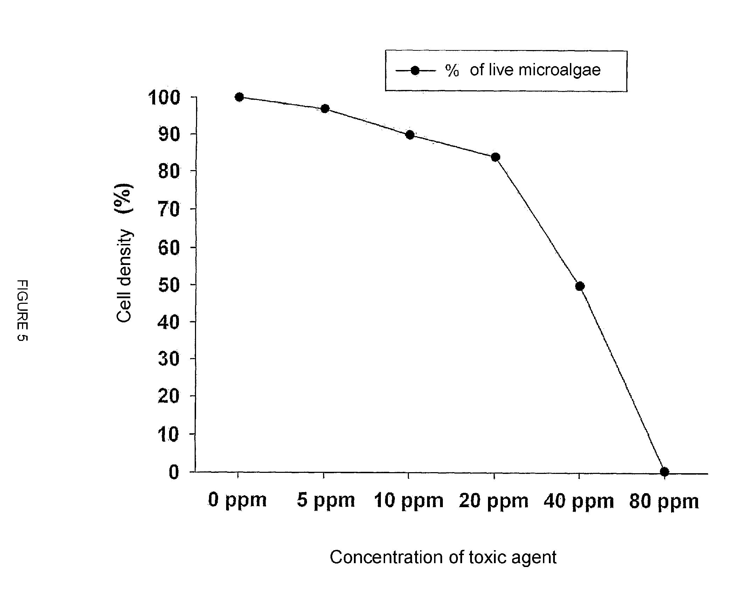

The exposure of the strain which is most sensitive (strain 20) to the toxic agent to increasing concentrations (0, 5, 10, 20, 40 mg L⁻¹) in BG-11 medium for 48 hours shows that the concentration inducing a lethal dose 100 is 80 mg L⁻¹ of toxic agent (see Figure 5).

The statistical analysis of the fluctuation with a low cell density (Set 1) and with a high cell density (Set 2) is summarized in Table 1. The Set 1 and Set 2 columns indicate the number of microalgae per tube of Set 1 and of Set 2. The fluctuation of Set 1 is very high whereas in Set 2 it is less than 2.

**Table 1**

| | | Set 1 | Set 2 |
|---|---|---|---|
| Number of tubes | | 100 | 50 |
| | | | |
| Number of microalgae per tube: | | | |
| | 0 | 54 | 0 |
| | <10³ | 20 | 0 |
| | 10³-10⁴ | 10 | 20 |
| | 10³-10⁵ | 15 | 30 |
| | >10⁵ | 1 | 0 |
| | | | |
| Variance/mean ratio | | >40 | 1.3 |
| Number of resistant microalgae | | 1 for every 4.410⁷ | |

Therefore, the variance/mean ratio is higher in Set 1 than in Set 2, and it can be asserted that the microalgae resistant to the toxic agent are generated from microalgae existing in the aquatic medium with mutations conferring resistance to said toxic agent.

The rate of division of the resistant microalga is (0.5±0.2), approximately one division every 2 days in the presence and in the absence of the toxic agent.

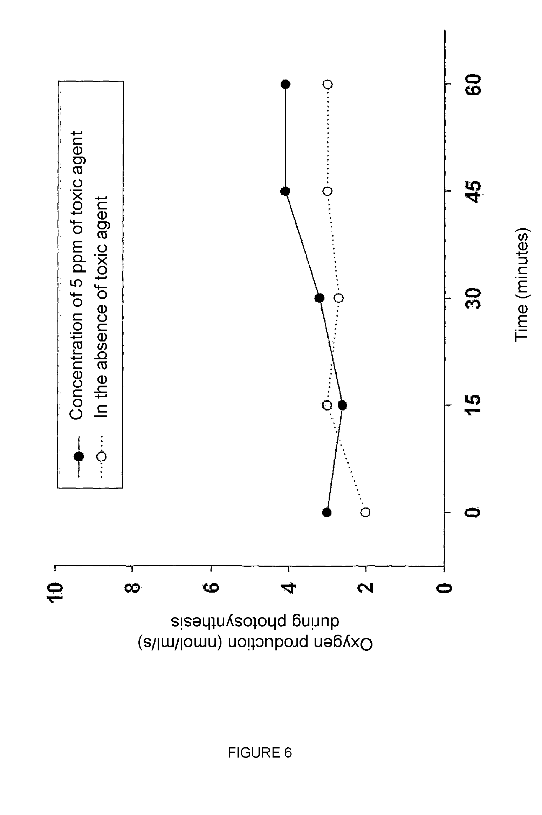

The oxygen production curves of microalgae resistant to the toxic agent are similar both in the presence of 5 ppm of the toxic agent and in the absence thereof (see Figure 6).

### Example 7

### Characterization of the joint response of sensitive and resistant microalgae in the absence, in the presence of the toxic agent and in the presence of an unspecific toxic agent.

### 7.1- In the absence of toxic agents

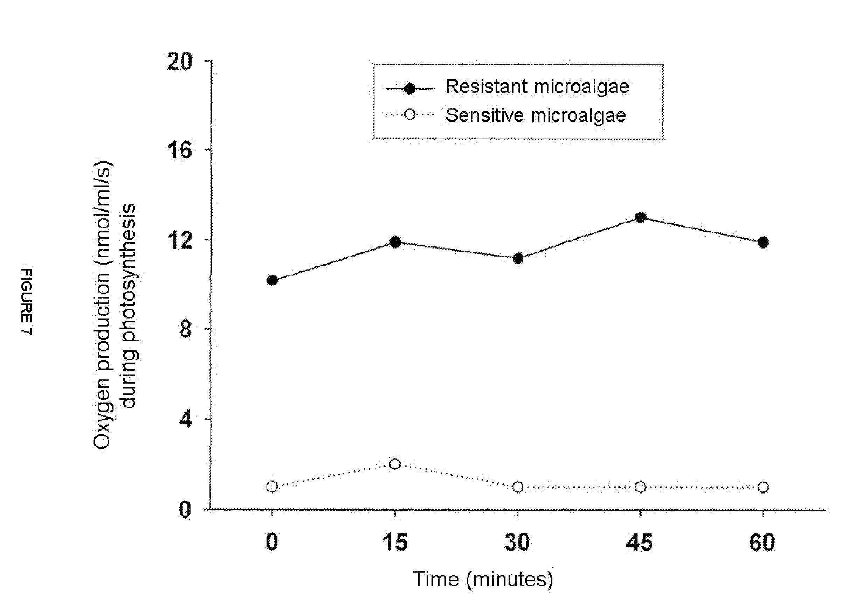

The oxygen production curve of sensitive and resistant microalgae in the absence of the toxic agent is different: the resistant microalgae produce less oxygen during photosynthesis than the sensitive microalgae, i.e., their behavior will be similar to that of the sensitive microalgae in the presence of toxic agent (see Figure 7).

### 7.2- In the presence of unspecific toxic agent

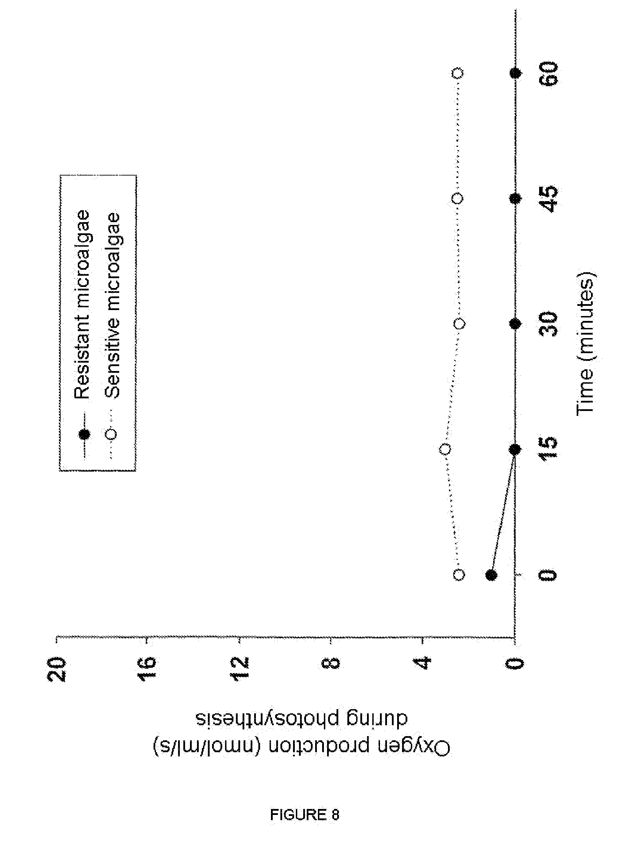

The oxygen production of sensitive and resistant microalgae in the presence of an unspecific toxic agent is similar (see Figure 8).

### 7.3- In the presence of the target toxic agent

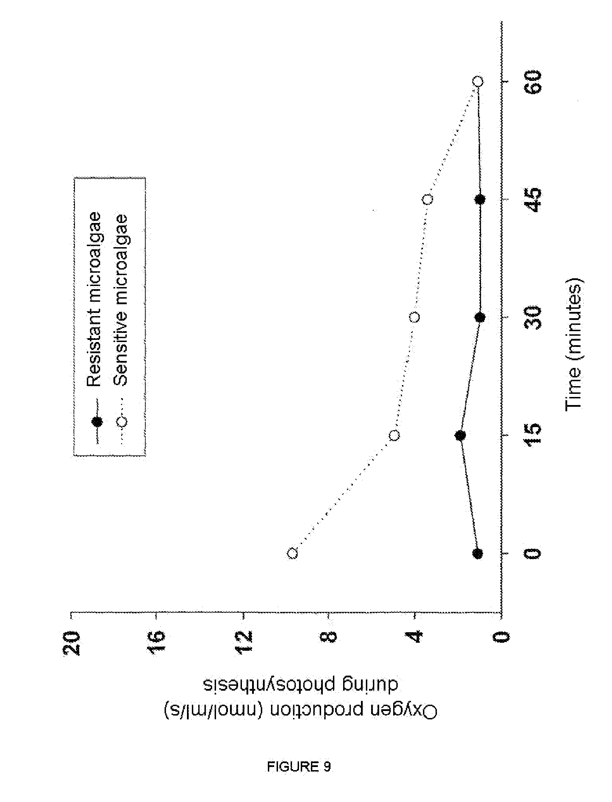

The oxygen production curve of sensitive and resistant microalgae in the presence of the toxic agent is different: the sensitive microalgae produce less oxygen during photosynthesis than the resistant microalgae, which allows determining the concentration of the target agent in the medium (Figure 9).

### Example 8

### Immobilization of microalgae sensitive and resistant to the toxic agent in an ImmobaSil three-dimensional membrane (Cellon, LU).

The microalgae sensitive and resistant to the toxic agent (for example, *Dictyosphaerium chlorelloides*) adhere to the membrane and occupy the pores thereof as can be observed in Figure 10. The viability of the cells in said membrane is 98%.

### Example 9

### Construction of the microalgal biosensor head.

The sensitive end of the microalgal biosensor is formed by two heads which are identical, except in that one contains microalgae sensitive to the target analyte (for example, *Dictyosphaerium chlorelloides*) and the other one contains the corresponding resistant clone thereof, both of them immobilized in a porous silicone membrane (such as for example, those of ImmobaSil type marketed by the company Cellon, Luxembourg).

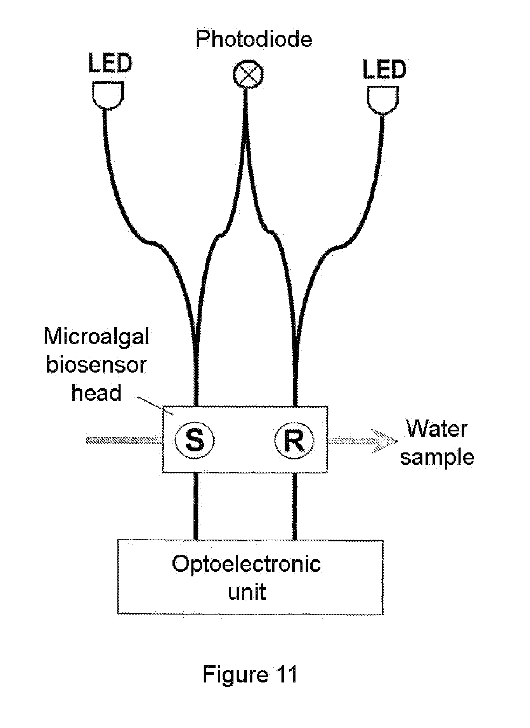

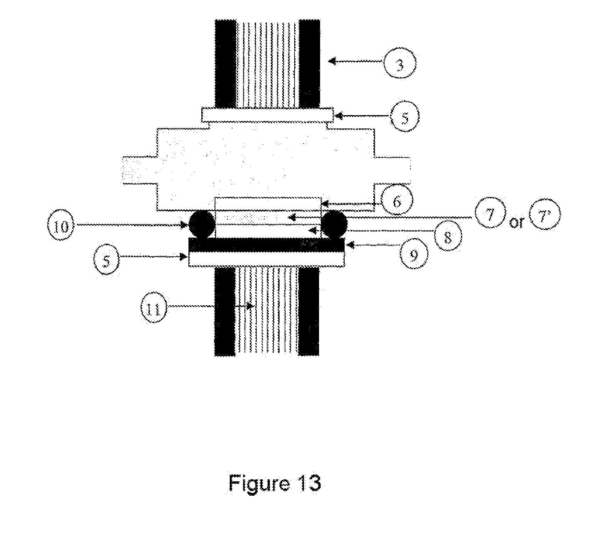

A diagram of each head is shown in Figure 13. The common end of a randomly bifurcated optical fiber (such as for example, those of optical glass or fused silica marketed by the company Vydas, United Kingdom) is arranged in a water-tight aluminium or stainless steel chamber. On the latter, there is arranged a thin transparent plastic or glass disk (5), and a thin luminescent film sensitive to molecular oxygen (9) (such as for example those described by Navarro-Villoslada et al. Anal. Chem. 2001, 73, 5150-5156) is arranged thereon. The thin film sensitive to oxygen is coated by a thin layer of polymer permeable to molecular oxygen but opaque to light (such as for example the black 732 silicone prepolymer manufactured and marketed by Dow-Corning, USA) (8). On the film sensitive to oxygen there is placed the porous silicone membrane disk containing the immobilized microalgae (7 or 7') (ImmobaSil, Cellon, LU) and, on the latter, there is placed a dialysis membrane (6) (such as for example Spectra/Por cellulose ester membranes marketed by the company Spectrum Europe BV, Holland). The water sample is made to flow on the latter by means of a multi-channel peristaltic pump (for example, from the brand Minipulse of Gilson, France). The sample chamber is closed by an aluminium or stainless steel cover provided with an optical plastic or glass windows (5). The second head is constructed in an identical manner. The heads are organized in the biosensor in the manner indicated in Figure 11. The biosensor additionally includes the elements indicated in Figure 12, i.e., a light source (1), a light detector (2), an optical glass trifurcated optical fiber (3), an entrance (4) and an exit of the sample (4'), a measurement optoelectronic unit with luminescent sensors (12), a blue band-pass optical filter (13) and a long-pass or cutoff optical filter (14).

The biosensor has an optical plastic or glass window through which the blue light from a light emitting diode (1) (such as, for example, the one having a maximum at 465 nm and marketed by the company Osram, Germany, with the code Golden Dragon LB W5SG-DYEZ-35) is passed. The light of the diode is lead to the optical window through one of the branches of a randomly trifurcated optical fiber made of glass, fused silica or plastic (such as the one solid by the company FiberGuide Industries, NJ, USA) (Figure 13). Finally, the common end of the trifurcated optical fiber is connected to a red-sensitive photodiode (2) capable of measuring the fluorescent emission of the chlorophyll contained in the immobilized microalgae, under the excitation of the blue diode. The signal of the photodiode is fed to the two-channel optoelectronic unit, which measures the oxygen sensors and controls the pumping of the sample (for example, the optoelectronic unit Optosen manufactured and marketed by Interlab IEC, Madrid).

## Claims

1. A specific biosensor for detecting a toxic compound comprising:
a) a first population of algae the oxygen production of which decreases in the presence of said compound,
b) a second population of algae the oxygen production of which does not vary substantially in the presence of said compound, of the same species as the first population and which has been obtained by means of a process comprising the steps of
(i) a first selection of spontaneous mutants viable to said compound by means of exposure to said toxic compound and
(ii) a second selection from the mutants obtained in step (i) in the presence of progressively higher concentrations of said toxic compound by means of at least one ratchet cycle
c) means for stimulating the photosynthetic activity of the two populations of algae and
d) means for detecting the oxygen produced by each of the two populations of algae
**characterized in that** the means for detecting the oxygen produced by each of the populations of algae comprise at least one compound the optical properties of which vary according to the concentration of oxygen in the medium and at least one optoelectric element suitable for detecting the optical properties of said compound.

2. A biosensor according to claim 1, wherein the first population of algae has been obtained from a wild-type algae strain by means of selecting spontaneous mutants showing an increased sensitivity to said toxic compound.

3. A biosensor according to claim 1 or 2, wherein the second population of microalgae is obtained from a wild-type algae strain which has been obtained by means of selecting spontaneous mutants showing an increased sensitivity to said toxic compound.

4. A biosensor according to any of the previous claims, **characterized in that** each of the populations of algae is immobilized in a support.

5. A biosensor according to claim 4, wherein the support is a three-dimensional matrix.

6. A biosensor according to any of the previous claims, **characterized in that** the two populations of algae are separated from the aqueous medium in which the measurement is made by means of a porous membrane impermeable to the microalgae but permeable to molecular oxygen.

7. A biosensor according to any of the previous claims, wherein the compound the optical properties of which vary according to the concentration of oxygen in the medium is impregnated in a membrane.

8. A biosensor according to any of the previous claims, wherein the compound the optical properties of which vary in the presence of oxygen in the medium is a complex of a transition metal and at least one diimine type ligand.

9. A biosensor according to claim 8, wherein the transition metal is selected from the group of ruthenium, osmium, rhenium, rhodium, platinum and indium.

10. A biosensor according to any of claims 8 or 9, wherein the diimine-type ligand is selected from the group of 2,2'-bipyridine and 1,10-phenanthroline and derivatives thereof with different degree of substitution.

11. A biosensor according to claim 10, wherein the compound the optical properties of which vary according to the concentration of oxygen is selected from the group of tris(1,10-phenanthroline)ruthenium(II), tris(4,7-diphenyl-1,10-phenanthroline)ruthenium(II), tris(2,2'-bipyridine)ruthenium(II), tris(4-phenyl-7-para-hexanolphenyl-1,10-phenanthroline)ruthenium(II) and (5-isothiocyanate-1,10-phenanthroline)bis(2,2'-bipyridine)ruthenium(II).

12. A biosensor according to any of claims 7 to 11, wherein the membrane impregnated with the compound the optical properties of which vary according to the concentration of oxygen in the medium is separated from the cells by an opaque membrane permeable to oxygen.

13. A biosensor according to any of the previous claims additionally comprising means for detecting the fluorescence of the chlorophyll of each of the populations of algae in response to the stimulus of the photosynthetic activity.

14. A biosensor according to any of the previous claims, wherein the species of alga forming the first and the second population of algae is *Dictyosphaerium chlorelloides*.

15. A biosensor according to any of the previous claims, wherein the compound which has been used to select the second population of algae is simazine.

16. Use of a biosensor according to claims 1 to 15 for detecting the presence of a toxic compound in a sample.

17. Use of a biosensor according to claims 1 to 15 for distinguishing the presence of the toxic compound causing a decrease of the oxygen production in the first population of algae from other toxic compounds in a sample.

18. Method for the detection of a toxic compound in a sample comprising the steps of
a) contacting the sample with a biosensor according to any of claims 1 to 15 wherein the second population of algae has been selected in the presence of said toxic compound and
b) detecting the production of light of each of the populations of cells in the biosensor in the presence of said sample
wherein a decrease in the production of light of the first population of cells with respect to the second population of cells indicates that the sample contains the toxic compound against which the cells of the second population have been selected and wherein a decrease in the production of light by the two populations of cells indicates that the sample contains a toxic compound different from the compound against which the first population of cells has been selected.

19. Method according to claim 18, wherein the total amount of algal biomass in each immobilized population in the absence of toxic agent is determined simultaneously and the values of variation in the production of light by the two populations of algae are standardized according to the amount of immobilized algal biomass of each population.

20. Method according to claim 19, wherein the determination of the amount of immobilized algal biomass is carried out by means of detecting the fluorescent emission of each population of algae in response to the stimulus of the photosynthetic activity.

21. A process for obtaining microalgae resistant to a toxic compound comprising the steps of
a) a first selection of viable spontaneous mutants in the presence of said toxic compound and
b) a second selection from the mutants isolated in step (a) in the presence of progressively higher concentrations of said toxic compound by means of at least one ratchet cycle.

22. A process according to claim 21, wherein the toxic compound obtained in the first and second step is simazine.

23. A process according to claim 21 or 22, wherein the alga which is subjected to selection is *Dictyosphaerium chlorelloides.*

24. An algae strain resistant to a toxic compound which can be obtained by means of a process according to any of claims 21 to 23.
